# EUROPEAN PATENT SPECIFICATION

(11) **EP 2 085 394 B1**
(45) Date of publication and mention of the grant of the patent: **06.06.2012**
(21) Application number: 09250251.7
(22) Date of filing: 30.01.2009
(51) Int. Cl.: C07D 403/12, C07D 413/12, C07D 417/12, A61K 31/4164, A61K 31/4184, A61P 31/04, A61P 33/02, C07D 233/92, A61K 31/423, A61K 31/428

(54) **Metronidazole derivatives as antiparasitic agents**
Metronidazol-Derivate als Antiparasitika
Dérivés de métronidazole en tant qu'agents antiparasites

(30) Priority: 03.02.2008 JO 4208
(43) Date of publication of application: 05.08.2009
(73) Proprietor: University of Jordan, 11942 Amman (JO)
(72) Inventor: Abu Shairah, Eman Ahmad Mohammad, Amman - Jordan 11942 (JO); Saadeh, Haythem Ali Mohammad, Amman - Jordan 11942 (JO); Mosleh, Ibrahim Mousa Ibrahim, Amman - Jordan 11942 (JO); Al Arif, Mikidad Tawfig Ayoub, Amman - Jordan 11942 (JO); Mubarak, Mohammad Suleiman (Al Haj M.), Amman - Jordan 11942 (JO)
(74) Representative: Simcox, Michael Thomas

(56) References cited:
- WO-A-2008/008480
- HAY, M.P.; LEE, H.H.; WILSON, W.R.; ROBERTS, P.B.; DENNY, W.A.: "Hypoxia-Selective Antitumor Agents. 10. Bis(nitroimidazoles) and Related Bis(nitroheterocycles): Development of Derivatives with Higher Rates of Metabolic Activation under Hypoxia and Improved Aqueous Solubility" JOURNAL OF MEDICINAL CHEMISTRY, vol. 38, 1995, pages 1928-1941, XP002524926
- BERTINARIA, M.; GALLI, U.; SORBA, G.; FRUTTERO, R.; GASCO, A.; BRENCIAGLIA, M.I.; SCALTRITO, M.M.; DUBINI, F.: "Synthesis and Anti-Helicobacter pylori Properties of NO-Donor/Metronidazole Hybrids and Related Compounds" DRUG DEVELOPMENT RESEARCH, vol. 60, 2003, pages 225-239, XP002524927
- BOIANI, L.; AGUIRRE, G.; GONZALEZ, M.; CERECETTO; CHIDICHIMO, A.; CAZZULO, J.J.; BERTINARIA, M.; GUGLIELMO, S.: "Furoxan-, alkylnitratederivatives and related compounds as anti-trypanosomatid-agents: Mechanism of action studies" BIORGANIC & MEDICINAL CHEMISTRY, vol. 16, 2008, pages 7900-7907, XP002524928
- KAJFEZ, F.; SUNJIC, V.; KOLBAH, D.; FAJDIGA, T.; OBLOBDZIJA: "1-Substitution in 2-Methyl-4(5)-nitroimidazole. I. Synthesis of Compounds with Potential Antitrichomonal Activity" JOURNAL OF MEDICINAL CHEMISTRY, vol. 11, 1968, pages 167-169, XP002524929
- FAUCI ET AL: "Harrison's Principles of Internal Medicine" 1 January 1998 (1998-01-01), 19980101 , XP002523866 * page 805; tables 129-3 * * page 943; tables 156-2 * * page 1172, column 1 - page 1173; tables 214-1 *

## Description

The present invention relates to the preparation of certain metronidazole derivatives and their use as antiparasitic agents and as antibacterial agents.

Metronidazole (2-(2- methyl- 5-nitro- 1*H*- imidazol- 1-yl) ethanol) has been used in the treatment of infections caused by anerobic bacteria and protozoa. A number of metronidazole derivatives have also been investigated. De Martino, G. et al., J. Med. Chem., 2005, 48, 4378 have prepared 1-[2-(diarylmethoxy)ethyl]-2-methyl-5-nitroimidazoles and studied their use as HIV-1 non-nucleoside reverse transcriptase inhibitors. Bertinaria, M. et al. Drug Dev. Res., 2003, 60, 225 prepared 2-(2-methyl-5-nitro-1H-imidazolyl)ethyl derivatives conjugated through an oxygen or an aminomethyl bridge with either a furoxan NO-donor moiety or a furazan substructure. All the compounds were evaluated *in vitro* for their activity against a number of Helicobacter pylori strains. The synthesised hybrids and their analogues exhibited good anti-H. pylori activity.

The following literature references describe metronidazle derivatives for treating antiparasitic infections:
1. Yuen, J. J. Heterocylic Chem. 2005, 29, 1021.
2. Hay, M. P., Anderson, R. F., Ferry, D. M., Wilson, W. R. and Denny, W. A. J. Med Chem., 2003, 46, 5533.
3. Günay, N. S., Çapan, G., Ulusoy, N., Ergenç, N., Ötük, G. and Kaya, D. Farmaco, 1999, 54, 826.
4. Goldman, P. and Wuest, J. D. J. Am. Chem. Soc., 1981, 103, 6224-6226.
5. Ross, W. J. and Jamieson, W. B. J. Med. Chem., 1973, 16, 347.

It has now been discovered that certain metronidazole derivatives, thereof, are useful as antiparasitic compounds, in particular against *Entamoeba histolytica* and *Giardia lamblia* and against *Leishmania donovani* and *Leishmania tropica* promastigotes.

Kaifez, F., Sunjic, V., Kolbah, D., Fajdiga, T., Oklobdzija, M. J. Med. Chem. 1968, II, 167 describes a phenyl-metronidazole compound and its use against *Trichomonas vaginalis*.

According to a first aspect of the invention there is provided a 2-methyl-5-nitro-imidazolyl compound with the general formula I. 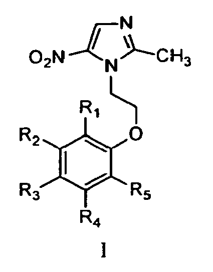 wherein:
one of the groups R₁, R₂, R₃, R₄, R₅ is an aldehyde; and each of the other groups R₁, R₂, R₃, R₄, R₅ is independently selected from hydrogen, alkyl, alkenyl, alkoxy, amino, alkylamino, dialkylamino, aryl, aralkyl, carboxyl, carboxylic ester, cyano, amido, alkylamido and heterocyclic groups;

### Compounds of Formula I

In one aspect the present invention relates to compounds of formula I: 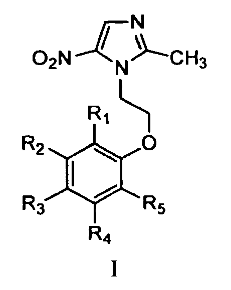 wherein one of the groups R₁, R₂, R₃, R₄, R₅ is an aldehyde.

Each of the other groups R₁, R₂, R₃, R₄, R₅ is independently selected from hydrogen, alkyl, alkenyl, alkoxy, amino, alkylamino, dialkylamino, aryl, aralkyl, carboxyl, cyano, amido, alkylamido and heterocyclic groups.

Preferably each of the other groups R₁, R₂, R₃, R₄, R₅ is independently selected from hydrogen, alkyl, alkenyl, alkoxy, amino, alkylamino, dialkylamino, carboxyl, cyano, amido, and alkylamido.

Preferably each of the other groups R₁, R₂, R₃, R₄, R₅ is independently selected from hydrogen, alkyl, alkoxy.

Preferably each of the other groups R₁, R₂, R₃, R₄, R₅ is independently selected from hydrogen and alkyl. Preferably each of the other groups R₁, R₂, R₃, R₄, R₅ is hydrogen.

In one aspect, preferably, each of R₁, R₂, R₄, R₅ is independently selected from hydrogen, alkyl, alkenyl, alkoxy, amino, alkylamino, dialkylamino, aryl, aralkyl, carboxyl, cyano, amido, alkylamido and heterocyclic groups. Preferably each of R₁, R₂, R₄, R₅ is independently selected from hydrogen, alkyl, alkenyl, alkoxy, amino, alkylamino, dialkylamino, carboxyl, cyano, amido, bromo, chloro, fluoro and alkylamido. Preferably each of the other groups R₁, R₂, R₄, R₅ is independently selected from hydrogen, alkyl, alkoxy, bromo, chloro and fluoro. Preferably each of the other groups R₁, R₂, R₄, R₅ is independently selected from hydrogen and alkyl. Preferably each of the other groups R₁, R₂, R₄, R₅ is hydrogen.

### Other Definitions

Any alkyl or alkenyl group, unless otherwise specified, may be linear or branched and may contain up to 10 carbon atoms. Preferred alkyl groups contain up to 6 carbon atoms. Preferably the alkyl groups contain up to 5 carbon atoms. More preferably the alkyl groups contain up to 4 carbon atoms. Preferred alkyl groups are methyl, ethyl, propyl, isopropyl, n-butyl, 2-methylpropyl and t-butyl. Preferred alkenyl groups include ethenyl (vinyl).

Alkoxy groups have the structure -O-alkyl. The alkyl portion of the alkoxy group may be any of the alkyl groups defined above. Preferred alkoxy groups are methoxy, ethoxy, propoxy, iso-propoxy, n-butoxy, 2-methylpropoxy and t-butoxy.

An aryl group may be any aromatic hydrocarbon group and may contain from 6 to 24 carbon atoms. Preferred aryl groups include phenyl, naphthyl, anthryl, phenanthryl and pyronyl groups, especially a phenyl or naphthyl, and particularly a phenyl, group.

A heterocyclic group may also be any monocyclic or polycyclic ring system which contains at least one heteroatom and may be unsaturated or partially or fully saturated. The term "heterocyclic" thus includes heteroaryl groups as well as non-aromatic heterocyclic groups. Preferably, a heterocyclic group is a 5- to 10-membered, ring system containing at least one heteroatom selected from oxygen, sulphur and nitrogen atoms.

Preferred heterocyclic groups include heteroaryl groups including pyridyl, pyrylium, thiopyrylium, pyrrolyl, furyl, thienyl, indolyl, isoindolyl, indolizinyl, imidazolyl, pyridonyl, pyronyl, pyrimidinyl, pyrazinyl, oxazolyl, thiazolyl, purinyl, quinolinyl, isoquinolinyl, quinoxalinyl, pyridazinyl, benzofuranyl, benzothiophenyl, chromenyl, 2-oxo-chromenyl, benzoxazolyl and acridinyl groups. Particularly preferred heteroaryl groups include indolyl, imidazolyl, pyridyl, thienyl and furyl groups, especially 3-indolyl, 4-imidazolyl, 2-pyridyl, 2-thienyl and 2-furyl groups.

The heterocyclic group 2-oxo-chromenyl has the following ring structure:

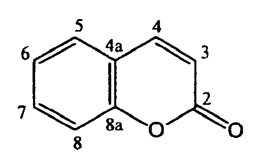

This heterocyclic group may be attached to the rest of the molecule by a bond to any of the 2, 3, 4, 5, 6, 7 or 8 positions as shown above. Preferably, such a heterocyclic group is attached to the rest of the molecule by a bond at the 4- or 6-position.

In addition to heteroaryl groups, preferred heterocyclic groups include pyronyl, piperidinyl, pyrrolidinyl, indolinyl, isoindolinyl, dioxanyl, piperazinyl, morpholinyl, thiomorpholinyl, morpholinosulphonyl, tetrahydroisoquinolinyl and tetrahydrofuranyl groups.

As used herein the terms "optionally subsituted" or "substituted derivative" refer to ring structures wherein one or more of the hydrogens in the basic ring structure are substituted with other groups. Suitable ring structures may be found in cyclic, aryl, heterocyclic and aromatic heterocyclic groups. Thus, for example, a phenyl ring may be defined as being optionally substituted with a fluoro group in the 2-postiton to result in a 2-fluorophenyl group (such a group is present in the compound prepared in Example 14). The preferred optionally substituted groups are, or substituted derivatives comprise, one or more groups independently selected from alkyl, alkenyl, alkoxy, amino, alkylamino, dialkylamino, carboxyl, carboxylic ester, cyano, amido, bromo, chloro, fluoro and alkylamido.

The preferred optionally substituted groups are, or substituted derivatives comprise, one or more groups independently selected from alkyl, alkoxy, carboxylic ester, fluoro, chloro and bromo.

Preferably the optionally substituted groups are, or substituted derivatives comprise, one or more groups independently selected from methyl, ethyl, propyl, iso-propyl, n-butyl, 2-methyl-propyl, t-butyl, fluoro, chloro, bromo, methoxy, ethoxy, propoxy, iso-propoxy, n-butoxy, 2-methyl-propoxy and t-butoxy, methyl ester, ethyl ester, propyl ester, iso-propyl ester, n-butyl ester, 2-methyl-propyl ester, t-butyl ester, fluoro, chloro and bromo.

Carboxylic esters have the structure -CO₂-alkyl. Preferably the or each carboxylic ester has the formula -CO₂R₁₈ wherein R₁₈ is an alkyl group as described herein. Preferably R₁₈ is a C1-C10 alkyl group; preferably R₁₈ is a C1-C6 alkyl group; preferably R₁₈ is a methyl ester, ethyl ester, propyl ester, iso-propyl ester, n-butyl ester, 2-methyl-propyl ester, t-butyl ester.

Furthermore, the present invention relates to a compound of formula IA (R₁ = R₂ = R₄ = R₅ = H, and R₃ = CH=O); This compound can be prepared according to Schemes 1.

An araalkyl group is an alkyl group as defined herein which is substituted with an aryl group as defined herein. Preferred araalkyl groups are phenyl-alkyl groups such as phenylmethyl, phenylethyl, and phenylpropyl.

### Uses and Other Aspects

The present invention also relates to the use of 2-methyl-5-nitro-imidazolyl compounds as described herein for use in medicine.

In a further aspect, the present invention relates to 2-methyl-5-nitro-imidazolyl compounds as described herein for use in the treatment of a parasitic infection. Preferably for use in the treatment of a parasitic infection caused by a parasite selected from *Entamoeba histolytica, Giardia lamblia* and pathogenic *Leishmania* species. Preferably the *Leishmania* species is selected from *Leishmania donovani* and *Leishmania tropica* promastigotes. Most preferably the compound is a compound with the formula IA, IIA or IIIA.

The invention also includes the use of a compound of the general formula I, II, or III as defined above for the manufacture of a medicament for use as antiprotozoal agent, especially against *Entamoeba histolytica* and *Giardia lamblia* parasites and against *Leishmania donovani* and *Leishmania tropica*

Compound IA, and reference compounds IIA, and IIIA have a potent lethal activity against the pathogenic parasites *Entamoeba histolytica* and *Giardia intestinalis.* The amount of compounds IIA and IIIA needed to kill half the population of the parasites (IC₅₀) was 20 times less than that of the original compound metronidazole, the standard drug used to treat infections with the two pathogenic parasites. Compound IA, on the other hand, was the most lethal to the two parasites and its IC₅₀ was 80 times less than that of the standard drug. The three derivatives, IA, IIA, and IIIA were also found to be equivalent in cytotoxicity to the standard drug when they were examined against Hep-2 and Vero cells. The three compounds also showed a lethal activity against *Leishmania donovani* and *Leishmania tropica* promastigotes.

In a further aspect, the present invention relates to 2-methyl-5-nitro-imidazolyl compounds as described herein for use in the treatment of a bacterial infection. Preferably in the treatment of a bacterial infection selected from *Clostridium, Bacteroides, Helicobacter, Fusobacterium*, and *Peptostreptococcus*.

A process for the preparation of a compound of 2-methyl-5-nitro-imidazolyl compound as described herein.

### Experimental

The following chemicals and materials employed in this study were used without further purification: 4-hydroxy benzaldehyde (Riedel-deHaen), metronidazole (Acros), 2-(2-methyl-5-1H-nitroimidazolyl)ethanamine (Acros), o-phenelenediamine (Fluka), 2-aminothiophenol (Fluka), 2-aminophenol (Riedel-deHaen AG), diisopropyl azodicarboxylate (Fluka), triethylamine (S.D. Fine-Chem Limitid), 3-bromo-5-clorothiophene-2-sulfonyl chloride (Aldrich), triphenylphosphin (Riedel-deHaen AG), column silica gel 60 (Fluka), silica gel plates ((Macherey-Nagel). Melting points (uncorrected) were determined on a Gallenkamp electrothermal melting temperature apparatus. NMR spectra were recorded on a Bruker DPX-300 instrument with TMS as internal reference. Electron Impact mass spectra (EIMS) were obtained with a Finnigan MAT TSQ-70 spectrometer at 70 eV; Elemental analyses were acquired with the aid of Eurovector Euro EA3000, CHNS-O elemental analyzer.

The invention is further illustrated by the following examples.

### Example 1

### Synthesis of compound IA

This example describes the synthesis of cmpound IA which was prepared according to the following procedure (Scheme 1):

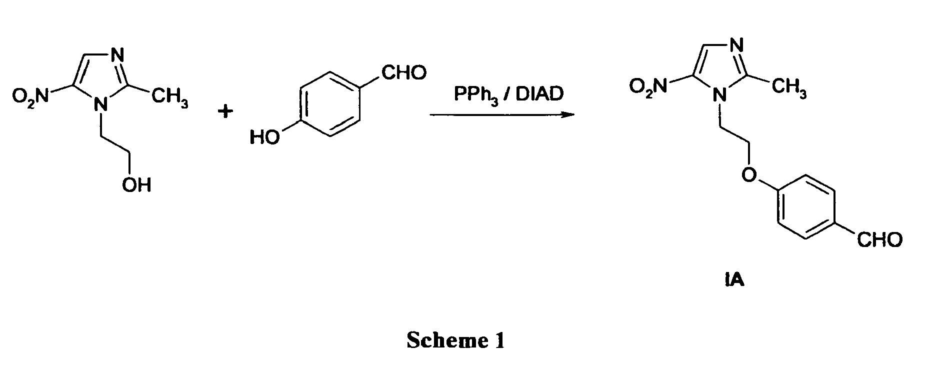

To a solution of metronidazole (4.62 g, 27 mmol), 4-hydroxy benzaldehyde (3.66 g, 30 mmol) and triphenyl phosphine, (8.91 g, 34 mmol) in dry THF (25 mL) at 0 °C, was added diisopropyl azodicarboxylate, DIAD, (6.87 g, 34 mmol) dropwise under N₂. The mixture was stirred for 1 hour and was then allowed to warm up to room temperature. Water was then added and the mixture was extracted with CHCl₃ (3 x 30 mL). The organic layer was dried over anhydrous Na₂SO₄ and concentrated under reduced pressure. The residue was purified by column chromatography with CHCl₃/ pet.ether (9: 1), to give yellow crystals (5.75 g,77%). Mp. 115-117 °C. ¹H-NMR (CDCl₃): δ 9.80 (s, 1H, *CHO*); 7.89 (s, 1H, ArH); 7.78 (d, 2H, *J* = 8.7 Hz, ArH); 6.90 (d, 2H, *J* = 8.7 Hz, ArH); 4.73 (t, 2H, *J*= 4.8 Hz); 4.48 (t, 2H, *J*= 4.8 Hz); 2.57 (s, 3H, *CH₃*). ¹³C-NMR (CDCl₃): δ 190.7 (*CHO*); 162.5 (C-4"); 151.7 (C-2), 138.4 (C-5); 133.4 (C-4); 132.1 (C-2", 6"); 130.7 (C-1"); 114.6 (C-3", 5"); 66.9 (C-1'); 45.8 (C-2'); 14.7 (*CH₃*).
EIMS (70 eV), *m*/*z* (rel. int.): 275 [M]⁺ (20), 258 (2), 245 (1); 229 (20), 201 (71), 174 (10), 154 (100), 95 (49). Anal. Calcd. for C₁₃H₁₃N₃O₄: C, 56.72; H, 4.76; N, 15.27.
Found: C, 57.06 H, 4.68; N, 15.24. IR: 3124, 2807, 2741, 1679, 1603, 1578, 1534, 1478, 1368, 1251, 1157, 1052, 901 cm⁻¹.

### Reference Example 2

Compound IIa was synthesized according to the following procedure outlined below (Scheme 2):

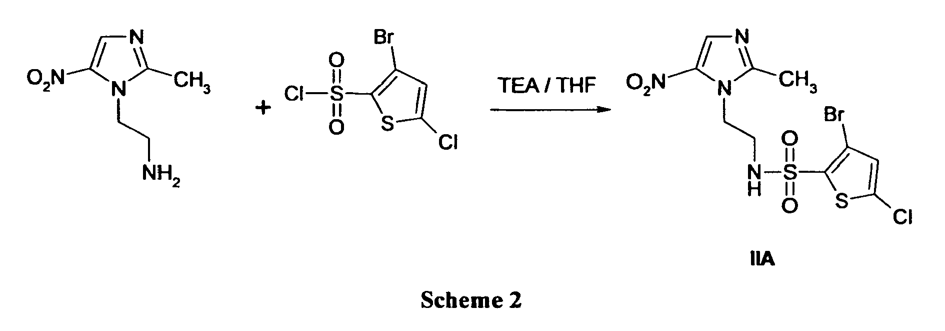

To a stirred solution of 2-(2-methyl-5-nitro-1*H*-imidazol-1-yl)ethanamine (0.31 g, 1.18 mmol) and triethylamine (Et₃N, 0.3 ml, 1.26 mmol) in CH₂Cl₂ (8 ml) at 0 °C, was added 3-bromo-5-chlorothiophene-2-sulfonyl chloride (0.34 g, 1.15 mmol) portion wise. After stirring the reaction mixture for 16 h at room temperature, the solvent was removed under reduced pressure. The residue was stirred with water and the solid was filtered and recrystallized from aqueous ethanol (0.26 g, 51%, mp. 197-198 °C). ¹H-NMR (DMSO):δ 2.43 (s, 3H, *CH₃),* 3.35 (m, 2H, C₁-H), 4.29 (t, 2H, *J* = 6.0 Hz, C₂-H), 7.44 (s, 1H, C₄-H), 7.97 (s, 1H, C₄-14), 8.65 (t, 1H, *J* = 6.0 Hz, *NH*). ¹³C-NMR: δ 14.7 (*CH₃*), 42.3 (C-1"), 46.4 (C-2"), 112.5 (C-3), 133.0 (C-4), 133.7 (C-4'), 135.0 (C-2), 135.5 (C-5), 138.8 (C-5'), 152.2 (C-2'). MS (C₁₀H₁₀N₄O₄S₂ClBr), *m*/*z* (% rel. int.): 431 [M]⁺ (traces), 384 (34), 260 (73), 196 (23), 123 (100). Anal. Calcd. for (C₁₀H₁₀N₄O₄S₂ClBr): C, 27.95; H, 2.35; N, 13.04; S, 14.92. Found: C, 28.01; H, 2.67; N, 13.10; S, 14.96. IR: 3106, 3004, 2798, 2727, 1533, 1499, 1487, 1403, 1340, 1263, 1188, 1160, 1097, 1016, 825 cm⁻¹.

### Reference Example 3

This compound was synthesized according to Scheme 3.

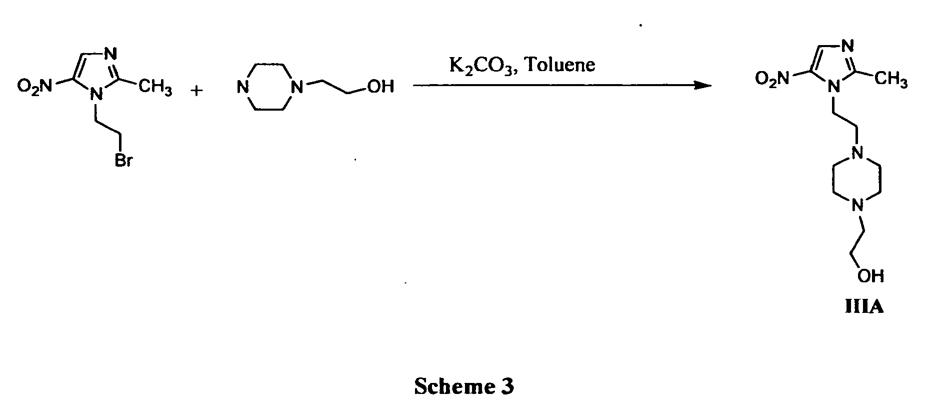

A mixture of 1-(2-bromoethyl)-2-methyl-5-nitroimidazole (0.3 g, 1.3 mmol), and 1-ethanol piperazine (0.17 g, 1.3 mmol), K₂CO₃ (0.21 g, 1.4 mmol) and Nal (.25 g, 1.4 mmol) in toluene (5 ml) was refluxed for 24 h. The reaction mixture was cooled, treated with water, then extracted with CHCl₃. The organic layer was dried (anhydrous Na₂SO₄) and concentrated under reduced pressure. The crude product was purified by silica gel plates using AcOEt / Hexane (3:1), to give compound IIIA as an oily product (0.11 g, 31%). ¹H-NMR (CDCl₃): δ 2.47 (s, 3H, *CH₃*), 2.55 (*br* t, 4H, C_{2",5"}-H, 2.61 (t, 2H, *J =* 4.7 Hz, C_{2"'}-H), 2.71 (*br* t, 4H, C_{3",4"}-H), 3.53 (t, 2H, *J=* 6.2 Hz, C₁-H), 3.58 (t, 2H, *J=* 4.7 Hz, C_{1"'}-H), 4.33 (t, 2H, *J =* 6.2 Hz, C₂-H), 4.88 *(br* s, 1H, *OH*), 7.90 (s, 1H, C_{4'}-H). ¹³C-NMR: δ 14.2 (*CH₃*), 43.99 (C-1"'), 52.8 (C-2",5"), 53.6(C-3",4"), 57.7 (C-2"'), 57.8 (C-1), 59.3 (C-2), 132.6 (C-4'), 138.7 (C-5'), 150.9 (C-2'). MS (C₁₂H₂₁N₅O₃), *m*/*z* (% rel. int.): 284 [M+1]⁺, (14); 283 [M⁺], (2); 252 (100), 235 (9), 206 (15).

### Reference Example 4

### 1-(2-Bromoethyl)-2-methyl-5-nitro-1-imidazole

To a mixture of metronidazole (3.42 g, 20 mmol) and carbon tetrabromide (8 g, 30 mmol) in dry THF (20 mL) was added triphenylphosphine (5.8 g, 21 mmol) at 0 °C in several portions over a 15-minute period and the mixture was stirred for 1.5 hour at room temperature. Water was then added and the mixture was extracted with CH₂Cl₂ (2 x 20 mL). The organic layer was dried over anhydrous Na₂SO₄ and concentrated under reduced pressure. The residue was purified using column chromatography with CH₂Cl₂ /hexane (3: 1), to give pale-yellow crystals. (54.26 g, 91%, mp. 81-82 °C); (Lit. mp = 80-81°C). ¹¹¹H-NMR (CDCL₃): δ 2.50 (s, 3H, CH₃), 3.64 (t, 2H, *J*= 6.1 Hz, C₁-H), 4.61 (t, 2H, *J* = 6.1 Hz, C₂-H), 7.90 (s, 1H, C₄-H). ¹³C-NMR: δ 14.03 (CH₃), 39.2 (C-1'), 44.6 (C-2'), 132.8 (C-4), 138.8 (C-5), 152.1 (C-2).

### Reference Example 5

### 2-{4-[2-(2-Methyl-5-nitro-1H-imidazol-1-yl)ethoxy]phenyl}-1H-beozimidazole (Ib)

A stirred mixture of 4-[2-(2-methyl-5-nitro-1*H*-imidazol-1-yl)ethoxy]benzaldehyde (0.69 g, 2.5 mmol) and *o*-phenylenediamine (0.27 g, 2.53 mmol) in DMSO (5 mL) was refluxed at 120 °C in an oil bath for 16 hours. The reaction mixture was cooled to room temperature, and water was then added. The product was extracted with AcOEt; the organic layer was dried over anhydrous Na₂SO₄ and concentrated under reduced pressure. The resulting product was purified on preparative silica gel plates using CHCl₃ / MeOH (9:1) to give orange crystals (0.61 g, 76%). mp. 253-255 °C).

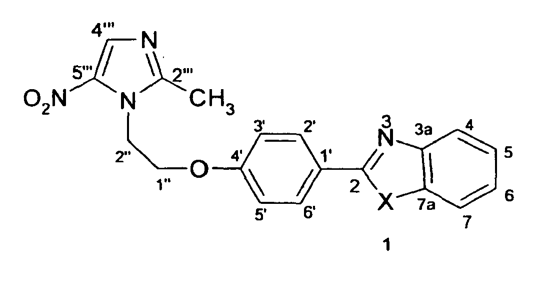

¹H-NMR DMSO): δ 2.52 (s, 1H, CH₃), 4.40 (t, 2H, *J* = 4.6 Hz, C_{1"}-H), 4.71 (t, 2H, *J =* 4.6 Hz, C_{2"}-H), 7.04 (d, 2H, *J =* 8.7 Hz, C_{3',5'}-H), 7.12 (dd, 2H, *J =* 2.9, 5.7 Hz, C_{4,7}-H,), 7.51 (dd, 2H, *J* = 2.9, 5.7 Hz, C_{5,6}-H), 8.01(s, 1H, C_{4"'}-H), 8.08 (d, 2H, *J* = 8.7 Hz, C_{2',6'}-H), 12.84 (s, 1H, *NH*). ¹³C-NMR: δ 14.7 (*CH₃*), 45.74 (C-2"), 67.0 (C-1"), 111.6 (C-7), 115.3 (C-3',5'), 118.9 (C-5), 121.9 (C-4), 122.6 (C-6), 123.7 (C-1'), 128.5 (C-2',6'), 133.4 (C-4"'), 135.5 (C-7a), 139.0 (C-5"'), 144.3 (C-3a), 151.6 (C-2"'), 152.3 (C-2), 159.7 (C-4'). EIMS (70 eV), *m*/*z* (rel. int.): 364 [M+1]⁺ (14), 363 [M]⁺, (88), 317 (2), 209 (22); 193 (12), 154 (100), 109 (23). Anal. Calcd. for (C₁₉H₁₇N₅O₃): C, 62.80 ; H, 4.72; N, 19.27. Found: C, 62.53; H, 4.88; N, 18.98. IR: 3407, 3188, 3135, 1611, 1531, 1498, 1475, 1429, 1367, 1292, 1253, 1189, 1068, 829, 736 cm⁻¹.

### Reference Example 6

### 2-{4-[2-(2-Methyl-5-nitro-1H-imidazol-1-yl)ethoxy]phenyl}-1,3-benzoxazole (Ic)

A stirred mixture of 4-[2-(2-methyl-5-nitro-1*H*-imidazol-1-yl)ethoxy]benzaldehyde (0.69 g, 2.5 mmol) and 2-aminophenol (0.27 g, 2.53 mmol) in nitrobenzene (5 ml) was refluxed at 200 °C in an oil bath for 16 h. After cooling the reaction to room temperature, water was added. The product was extracted by AcOEt, the organic layer was dried (anhydrous Na₂SO₄) and concentrated under reduced pressure. The crude product was purified on preparative silica gel plates using CHCl₃ / MeOH (9:1) to give orange crystals (0.81 g , 88%, mp. 205-207 °C). ¹H-NMR (CDCl₃): δ 2.63 (s, 3H, *CH₃*), 4.39 (t, 2H, *J* = 4.9 Hz, C_{1"}-H), 4.74 (t, 2H, *J* = 4.9 Hz, C_{2"}-H), 6.93 (d, 2H, *J* = 8.8 Hz, C_{3',5'}-H), 7.30 (m, 2H *overlapping*, C_{4,7}-H), 7.54 (dd, 1H, *J* = 1.7, 5.9 Hz, C₅-H), 7.72 (dd, 1H, *J =* 1.7, 5.9 Hz, C₆-H), 7.97 (s, 1H, C_{4"'}-H), 8.17 (d, 2H, *J =* 8.8 Hz, C_{2',6'}-H). ¹³C-NMR: δ 14.8 (*CH₃*), 45.9 (C-2"), 66.8 (C-1"), 110.5 (C-7), 114.7 (C-3',5'), 119.8 (C-4), 120.8 (C-1'), 124.6 (C-5), 124.9 (C-6), 129.5 (C-2',6'), 133.4 (C-4"'), 138.4 (C-5'''), 142.2 (C-3a), 150.7 (C-7a), 151.7 (C-2"'), 160.3 (C-2), 162.7 (C-4'). MS (70 eV) (C₁₉H₁₆N₄O₄), *m*/*z* (% rel. int.): 364 [M]⁺ (77); (88); 318 (6); 210 (21); 194 (14), 154 (100), 109 (31). Anal. Calcd. for (C₁₉H₁₆N₄O₄): C, 62.63 ; H, 4.43; N, 15.38 . Found: C, 62.81; H, 4.42; N, 15.68 . IR: 3118,2997, 1619, 1584, 1521, 1501, 1465, 1428, 1360, 1251, 1181, 1059, 831, 745 cm⁻¹.

### Reference Example 7

### 2-{4-[2-(2-Methyl-5-nitro-1H-imidazol-1-yl)ethoxy]phenyl}-1,3-benzothiazole (Id)

A stirred mixture of 4-[2-(2-methyl-5-nitro-1*H*-imidazol-1-yl)ethoxy]benzaldehyde (0.69 g, 2.5 mmol) and 2-aminothiophenol (0.32 g, 2.53 mmol) in dimethylsulfoxide (DMSO, 5 ml) was refluxed at 120 °C in an oil bath for 16 h. The reaction mixture was cooled to room temperature, and the precipitate was filtered, washed with H₂O and dried. The crud product was recrystallized from CHCl₃ and pet.ether to give white crystals (0.77 g, 80%, mp. 198-200 °C). ¹H-NMR (CDCl₃): δ 2.6 (s, 3H, *CH₃*), 4.35 (t, 2H, *J* = 4.8 Hz, C_{1"}-H), 4.69 (t, 2H, *J =* 4.8 Hz, C_{2"}-H), 6.88 (d, 2H, *J =* 8.8 Hz, C_{3',5'}-H), 7.33 (ddd, 1H, C₆-H), 7.44 (ddd, 1H, C₅-H), 7.84 (d, 1H, *J* = 8.0 Hz, C₄-H ), 7.97 (s, 1H, C_{4"'}-H), 7.99 (d, 2H, *J* = 8.8 Hz, C_{2',6'}-H), 8.02 (d, 1H, *J=* 8.0, C₇-H). ¹³C-NMR: δ 14.7 (*CH₃*), 45.9 (C-2"), 66.8 (C-1"), 114.6 (C-3',5'), 121.6 (C-4), 122.9 (C-7), 125.0 (C-6), 126.3 (C-5), 127.4 (C-1'), 129.2 (C-2',6'), 133.4 (C-4'''), 134.9 (C-3a), 138.9 (C-5"'), 151.8 (C-2"'), 154.2 (C-7a), 159.9 (C-4'), 167.4 (C-2). MS (70 eV) (C₁₉H₁₆N₄O₃S), *m*/*z* (% rel. int.): 380 [M]⁺ (75); 334 (5), 227 (36); 154 (100), 108 (37). Anal. Calcd. for C₁₉H₁₆N₄O₃S: C, 59.99; H, 4.24; N, 14.73. Found: C, 59.64; H, 4.17; N, 14.63. IR: 3131, 2939, 1608, 1522, 1487, 1467, 1435, 1366, 1248, 1187, 1062, 956, 827, 760 cm⁻¹.

### Reference Example 8

### 7-Methoxy-4-methyl-N-[2-(2-methyl-5-nitro-1H-imidazol-1-yl)ethyl]-2-oxo-2H-chromene-6-sulfonamide (IIb)

To a stirred solution of 2-(2-methyl-5-nitro-1*H*-imidazol-1-yl)ethanamine (0.31 g, 1.18 mmol) and triethylamine (Et₃N, 0.3 ml, 1.26 mmol) in CH₂Cl₂ (8 ml) at 0 °C, was added 7-methoxy-4-methyl-2-oxo-2*H*-chromene-6-sulfonyl chloride (0.29 g, 1.15 mmol) portion wise. After stirring the reaction mixture for 16 h at room temperature, the solvent was then removed under reduced pressure. The residue was stirred with water and the solid was filtered, washed with ethyl ether and recrystallized from aqueous ethanol giving white crystals (0.36 g , 72%, mp. 260-263 °C).

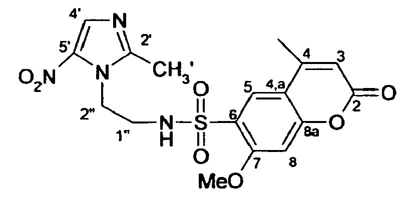

¹H-NMR (DMSO): δ 2.38 (s, 3H, *CH₃*), 2.41 (s, 3H, *CH₃'),* 3.23 (m, 2H, C_{1"}-H), 3.92 (s, 3H, *OMe*), 4.24 (t, 2H, *J*= 5.5Hz, C_{2"}-H), 6.29 (s, 1H, C₃-H), 7.16 (s, 1H, C₈-H), 7.72 (*br* s, 1H, *NH*), 7.83 (s, 1H, C₅-H), 7.87 (s, 1H, C₄-H). ¹³C-NMR: δ 14.6 (*CH₃'*), 18.4 (*CH₃*), 42.1 (C-1"), 46.6 (C-2"), 57.6 (*OMe*), 101.7 (C-8), 112.7 (C-4a), 112.8 (C-3), 125.1 (C-6), 126.9 (C-5), 133.6 (C-4'), 138.7 (C-5'), 152.2 (C-2'), 153.4 (C-4), 157.8 (C-8a), 159.2 (C-2), 159.8 (C-7). MS (C₁₇H₁₈N₄O₇S), *m*/*z* (% rel. int.): 422 [M]⁺ (traces), 376 (9), 253 (40), 189 (7),123 (100). Anal. Calcd. for (C₁₇H₁₈N₄O₇S): C, 48.34; H, 4.30; N, 13.26; S, 7.59. Found: C, 48.51; H, 4.71; N, 13.29; S, 7.53. IR: 3357, 3100, 3030, 2991, 2946, 1734, 1607, 1525, 1466, 1366, 1321, 1151, 1061, 991 cm⁻¹.

### Reference Example 9

### 4,7-Dimethyl-N-[2-(2-methyl-5-nitro-1H-imidazol-1-yl)ethyl]-2-oxo-2H-chromene-6-sulfonamide (IIc)

This compound was prepared from 2-(2-methyl-5-nitro-1*H*-imidazol-1-yl)ethanamine (0.31g, 1.18 mmol) and 4,7-dimethyl-2-oxo-2*H*-chromene-6-sulfonyl chloride (0.28 g, 1.15 mmol) following similar procedure and experimental conditions described above. The solid recrystallized from aqueous ethanol giving white crystals( 0.41 g, 86%, mp. 245-247 °C). ¹H-NMR (DMSO): δ 2.39 (d, 3H, *J* = 0.7 Hz, *CH₃"*), 2.41 (s, 3H, *CH₃'),* 2.52 (s, 3H, *Me*), 3.22 (m, 2H, C_{1"}-H), 4.26 (t, 2H, *J*= 6.0 Hz, C_{2"}-H), 6.43 (d, 1H, *J=* 0.7 Hz, C₃-H), 7.36 (s, 1H, C₈-H), 7.83 (s, 1H, C₅-H), 7.96 (s, 1H, C_{4'}-H), 8.14 (t, 1H, *J* = 6.0 Hz, *NH*). ¹³C-NMR: δ 14.6 (*CH₃'*), 18.3 (*CH₃")*, 20.3 *(Me),* 41.8 (C-1"), 46.4 (C-2"), 115.2 (C-3), 117.8 (C-4a), 120.7 (C-8), 126.2 (C-5), 133.7 (C-4'), 134.8 (C-6), 138.7 (C-5'), 141.3 (C-7), 152.1 (C-2'), 153.1 (C-4), 155.3 (C-8a), 159.6 (C-2). MS (C₁₇H₁₈N₄O₆S), *m*/*z* (% rel. int.): 405 [M⁺-1] (2), 360 (40), 237 (75), 173 (85), 123 (100). Anal. Calcd. for (C₁₇H₁₈N₄O₆S): C, 50.24; H, 4.46; N, 13.79; S, 7.89. Found: C, 50.09; H, 4.66; N, 13.82; S, 7.91. IR: 3136, 2980, 2872, 1737, 1624, 1542, 1483, 1426, 1363, 1258, 1146, 1042, 904, 823 cm⁻¹.

### Reference Example 10

### 7-methoxy-4-({[2-(2-methyl-5-nitro-1H-imidazol-1-yl)ethyl]amino}methyl)-2H-chromen-2-one (IId)

To a stirred solution of 2-(2-methyl-5-nitroimidazolyl)ethanamine (0.30 g, 1.15 mmol) and triethylamine (Et₃N, 0.3 ml, 1.26 mmol) in DMF (4 ml), was added 4-bromomethyl-7-methoxycoumarin (0.31 g, 1.15 mmol) portionwise. The resulting solution was stirred overnight, and then the solvent was removed under reduced pressure. The residue was stirred with water and the solid was filtered and purified on preparative silica gel plates using AcOEt / hexane (8:2) to give white crystals (0.27 g, 65%, mp 146-148 °C).

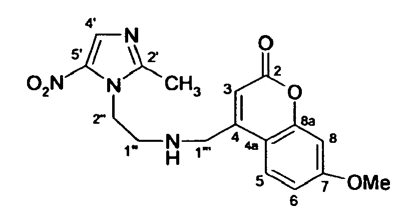

¹H-NMR (DMSO): δ 2.42 (s, 3H, *CH₃*), 2.60 *(br* s, 1H, *NH*), 2.88 (m, 2H, C_{1"}-H), 3.80 (s, 3H, *OMe*), 3.84 (br s, 2H, C_{1"'}-H), 4.34 (t, 2H, *J* = 5.8 Hz, C_{2"}-H), 6,09 (s, 1H, C₃-H), 6.85 (dd, 1H, *J* = 8.8, 2.5 Hz, C₆-H), 6.93 (d, 1H, *J* = 2.5 Hz, C₈-H), 7.59 (d, 1H, *J* = 8.8 Hz, C₅-H), 7.97 (s, 1H, C_{4'}-H). ¹³C-NMR: δ 14.7 (*CH₃*), 46.4 (C-1"), 48.7 (C-1"'), 48.8 (C-2"), 56.4 (*OMe*), 101.2 (C-8), 109.3 (C-3), 112.1 (C-4a), 112.5 (C-6), 126.2 (C-5), 133.5 (C-4'), 138.9 (C-5'), 152.1 (C-2'), 155.3 (C-4), 155.4 (C-8a), 160.9 (C-2), 162.6 (C-7). MS (C₁₇H₁₈N₄O₅), *m*/*z* (% rel. int.): 358 [M]⁺ (traces), 312 (30), 190 (29), 123 (77), 53(100). Anal. Calcd. for (C₁₇H₁₈N₄O₅): C, 56.98; H, 5.06; N, 15.63. Found: C, 56.79; H, 5.57; N, 15.68. IR: 3412, 3300, 3127, 2846, 1708, 1617, 1523, 1454, 1362, 1298, 1261, 1184, 830 cm⁻¹.

### Reference Example 11

### 7-methyl-4-({[2-(2-methyl-5-nitro-1H-imidazol-1-yl)ethyl]amino}methyl)-2H-chromen-2-one (IIe)

This compound was prepared from 2-(2-methyl-5-nitro-1*H*-imidazol-1-yl)ethanamine (0.30 g, 1.15 mmol) and 4-bromomethyl-7-methycoumarin (0.34 g, 1.15 mmol) by following similar procedure and experimental conditions described above. The crud product was purified on silica gel plates using AcOEt / hexane (9:1) to give white crystals (0.21 g, 53%, mp. 181-183 °C). ¹H-NMR (DMSO): δ 2.36 (s, 3H, *Me*), 2.43 (s, 3H, *CH₃*), 2.58 (*br* s, 1H, *NH*), 2.87 (m, 2H, C_{1"}-H), 3.79 (d, 2H, *J=* 3.3 Hz, C_{1"'}-H), 4.33 (t, 2H, *J* = 5.9 Hz, C_{2"}-H), 6,18 (s, 1H, C₃-H), 7.15 (d, 1H, *J* = 8.1, C₅-H), 7.21 (s, I H, C₈-H), 7.58 (d, 1H, *J =* 8.1 Hz, C₆-H), 7.95 (s, 1H, C_{4'}-H). ¹³C-NMR: δ 14.6 (*CH₃*), 21.5 (*Me*), 46.4 (C-1"), 48.69 (C-1"'), 48.72 (C-2"), 111.5 (C-3), 116.3 (C-8), 117.0 (C-4a), 124.9 (C-6), 125.7 (C-5), 133.5 (C-4'), 138.9 (C-5'), 143.0 (C-7), 152.0 (C-2'), 153.6 (C-4), 155.2 (C-8a), 160.6 (C-2). MS (C₁₇H₁₈N₄O₄), *m*/*z* (% rel. int.): 342 [M]⁺ (4), 296 (100), 174 (77), 145 (97), 53 (98). Anal. Calcd. for (C₁₇H₁₈N₄O₄): C, 59.64; H, 5.30; N, 16.37. Found: C, 59.78; H, 5.80; N, 16.15 .IR: 3309, 3123, 2846, 1709, 1622, 1525, 1456, 1367, 1262, 1183, 1149, 862, 788 cm⁻¹.

### Reference Example 12

### Ethyl-6-methoxy-3-{[2-(2-methyl-5-nitro-1H-imidazol-1-yl)ethyl-amino]methyl}-1-benzofuran-2-carboxylate (13f) (IIf)

The title compound was prepared from 2-(2-methyl-5-nitro-1*H*-imidazol-1-yl)ethanamine (0.30 g, 1.15 mmol) and 3-(bromomethyl)-6-methoxy-1-benzofuran-2-carboxylate (0.34 g, 1.15 mmol) by following similar procedure and experimental conditions described above. The solid was purified by using silica gel plates AcOEt / hexane (8:2) to give orange crystals (0.25 g, 54%, mp. 106-108 °C). 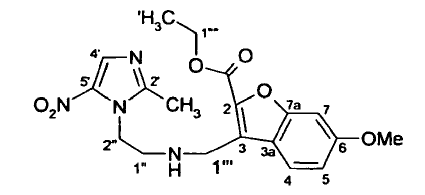 ¹H-NMR (CDCl₃): δ 1.29 (t, 3H, *J =* 7.1 Hz, *CH₃'*), 2.38 (s, 3H, *CH₃*), 2.46 (*br* s, 1H, *NH*), 2.85 (*br* s, 2H, C_{1"}-H), 3.36 *(br* s, 2H, C_{1"}-H), 3.79 (s, 3H, *OMe),* 4.13 (t, 2H, *J* = 6.0 Hz, C_{2"}-H), 4.29 (q, 2H, *J* = 7.1 Hz, C_{1""}-H), 6.89 (dd, 1H, *J* = 8.7,1.8 Hz, C₅-H), 7.23 (d, 1H, *J* = 1.8 Hz, C₇-H), 7.67 (d, 1H, *J* = 8.7 Hz, C₄-H), 7.93 (s, 1H, C_{4'}-H). ¹³C-NMR: 14.5 (*CH₃'*), 14.6 (*CH₃*), 42.3 (C-1"), 45.9 (C-1"'), 48.1 (C2"), 56.2 (*OMe*), 61.4 (C-1""), 96.1 (C-7), 113.9 (C-5), 121.1 (C-3a), 123.4 (C-3), 133.5 (C-4'), 138.8 (C-5'), 140.7 (C-2), 152.1 (C-2'), 155.73 (C-7a), 155.71 (C-3), 159.8 (*C*=*O*), 160.9 (C-6). MS (C₁₉H₂₂N₄O₆), *m*/*z* (% rel. int.): 403 [M]⁺ (traces), 356 (80), 205 (100), 123 (64). Anal. Calcd. for (C₁₉H₂₂N₄O₆): C, 56.71; H, 5.51; N, 13.92. Found: C, 56.29; H, 6.09; N, 13.64. IR: 3317, 3118, 3067, 2990, 2966, 2838, 1722, 1688, 1622, 1587, 1518, 1447, 1359, 1265, 1182, 1119, 820, 772 cm⁻¹.

### Reference Example 13

### 1-(2-Methoxyphenyl)-2-methyl-4-[2-(2-methyl-5-nitro-1H-imidazol-1-yl)ethyl]-piperazine (IIIb)

This compound was prepared from 1-(2-bromoethyl)-2-methyl-5-nitroimidazole (0.30 g, 1.3 mmol) and 3-methoxyphenyl-2-methyl piperazine (0.27 g, 1.3 mmol) following similar procedure and experimental conditions described above for. The resulting product was purified on silica gel plates using AcOEt / Hexane (3:1) to give oily product (0.28 g, 60%). ¹H-NMR (CDCl₃): δ 0.94 (d, 3H, *J=* 6.5 Hz, *Me*), 2.49 (s, 3H, *CH₃),* 2.55 (*br* t, 2H, C_{4"}-H), 2.65 (t, 2H, *J* = 6.4 Hz, C_{2"'}-H), 2.82 (*br* t, 2H, C_{3"}-H ), 3.04 (m, 1H, C_{2"}-H), 3.21 (m, 2H, C_{5"}-H), 3.72 (s, 3H, *OMe),* 4.39 (t, 2H, *J =* 6.4 Hz, C_{1"}'-H), 6.35 (*br* s, 1H, C₄-H), 6.38 (*br* s, 1H, C₆-H), 6.47 (*br* d, 1H, C₂-H), 7.1 (m, 1H, , C₅-H), 7.89 (s, 1H, C_{4'}-H). ¹³C-NMR: δ 13.1 (*Me*), 14.4 (*CH₃*), 44.0 (C-1"'), 44.5 (C-5"), 51.3 (C-2"), 53.8 (C-4"), 55.2 (*OMe*), 58.0 (C-2'''), 59.8 (C-3"), 103.5 (C-2), 104.6 (C-4), 109.8 (C-6), 129.8 (C-5), 132.9 (C-4'), 138.7 (C-5'), 150.6 (C-2'), 151,2 (C-1), 160.6 (C-3). MS (C₁₈H₂₉N₅O₃), *m*/*z* (% rel. int.): 359 [M]⁺ (96), 314 (14), 149 (98), 134 (100), 106 (77). IR: 2940, 2850, 2179, 1650, 1525, 1595, 1452, 1365, 1262, 1183, 1148, 1042, 1862, 730 cm⁻¹.

### Reference Example 14

### 1-(2-Fluorophenyl)-4-[2-(2-methyl-5-nitro-1H-imidazol-1-yl)ethyl]piperazine (IIIc)

A mixture of 1-(2-bromoethyl)-2-methyl-5-nitroimidazole (0.3 g, 1.3 mmol), 2-fluorophenyl piperazine (0.23 g, 1.3 mmol), K₂CO₃ (0.21 g, 1.4 mmol) and NaI (.25 g, 1.4 mmol) in toluene (5 ml) was refluxed for 24 h. The reaction mixture was cooled, treated with water, and then extracted with CHCl₃. The organic layer was dried (anhydrous Na₂SO₄) and concentrated under reduced pressure. The crude product was purified by silica gel plates using AcOEt / Hexane (3:1), to give white crystals (0.20 g, 46%, mp. 58-61 °C). 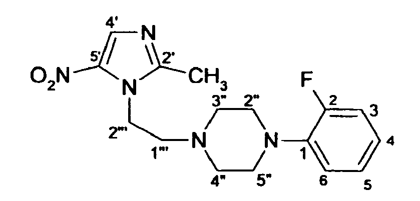 ¹H-NMR (CDCL₃): δ 2.49 (s, 3H, *CH₃*), 2.63 (t, 4H, *J*= 4.8 Hz, C_{3",4"}-H), 2.69 (t, 2H, *J* = 6.3 Hz, C_{2"'}-H), 3.02 (t, 4H, *J=* 4.8 Hz, C_{2",5"}-H), 4.39 (t, 2H, *J*= 6.3 Hz, C_{1"'}-H), 7.02 (m, 4H, C_{3,4,5,6}-H), 7.90 (s, 1H, C_{4'}-H). ¹³C-NMR: δ 14.4 *(CH₃),* 44.0 (C-1"'), 50.5 (d, ⁴*J*_{C-F} = 3,2 Hz, C-5",2"), 53.8 (C-3",6"), 57.9 (C-2"'), 116.3 (d, ²*J*_{C-F} = 20.55 Hz, C-3), 119.0 (d, ³*J*_{C-F} = 2,9 Hz, C-6), 122.7 (d, ³*J*_{C-F} = 7.9 Hz, C-4), 124.5 (d, ⁴*J*_{C-F} = 3,5 Hz, C-5), 132.8 (C-4'), 138.8 (C-5'), 138.9 (d, ²*J*_{C-F} = 9.5 Hz, C-1), 150.5 (C-2'), 154.3 (d, ¹*J*_{C-F} = 244 Hz, C-2). MS (C₁₆H₂₀N₅O₂F), *m*/*z* (% rel. int.): 333 [M]⁺ (33), 287 (36), 193 (100), 163 (9), 150 (78), 123 (53), 95 (43). Anal. Calcd. for (C₁₆H₂₀N₅O₂F): C, 57.65; H, 6.05; N, 21.01. Found: C, 57.51; H, 6.32; N, 21.03. IR: 3443, 2946, 2825, 1611, 1526, 1505, 1465, 1380, 1359, 1260, 1185, 1146, 752 cm⁻¹.

### Example 15

### Antiparasitic Activity

The following literature references describe the method used for testing and determining the antiparasitic acticvities of metronidazle derivatives.
**1.** Clark, C. G. and Diamond, L. S. Clin Microbiol Rev, 2002, 15, 329-341.
**2.** Burleson, F. G., Chambers, T. M, Wiedbrauk, D. L., Virology: a laboratory manual, 1992, London: Academic Press.
**3.** Keene, A. T., Harris, A., Phillipson, J. D, Warhurst, D. C., Planta Med, 1986, 52, 278-85 .
**4.** Neal, R. A. Arch Invest Med (Mex), 1978, 9, 387-92.

### Test organisms

*Entamoeba histolytica* HK-9 strain (ATCC number 30015) was cultured in LYI-S-2 medium supplemented with antibiotics. *Giardia intestinalis* WB strain (ATCC number 30957) was grown in a modified YI-S medium with antibiotics. Both parasites were cultivated in 15-mL screw-capped borosilicate glass tubes containing 13 mL medium. The tubes were incubated on a 15° horizontal slant at (36-37) °C. Culture maintenance and subculturing was performed as described by Clark and Diamond. *Entamoeba* and *Giardia* were harvested from confluent cultures by chilling of the tubes on ice for 5-10 min to detach cells, followed by centrifugation at 800 x g for 5 min. *Leishmania donovani* and *L. tropica* promastigotes were cultured in Schneider's medium containing 20 % foetal calf serum and antibiotics at 24°C. Stationary phase promastigotes were harvested by centrifugation at 1000 x g for 10 min.

### Antiamoebic and antigiardial activity

The antiamoebic and antigiardial activities of metronidazole-derivatives were tested using the tube method according to published procedures with some modifications. Eight milligrams of a test compound or metronidazole were dissolved in 10 µL of dimethyl sulfoxide (DMSO) and completed with 10 mL growth medium. The solutions were filter sterilized using 0.22 µm syringe filters and the appropriate volumes of the solutions were taken to prepare the concentrations of each compound in 15-mL screw-capped borosilicate glass tubes. For each compound, concentrations of 60, 30, 15, 7.5, 3.5, 1.7, 0.8, 0.4, 0.2, 0.1, 0.05, 0.02, 0.01, 0.005 µg/mL medium were prepared in a final volume of 15 mL to exclude air from the tube. For more accurate determination of the biologically active concentration of the compound, other concentrations around the above mentioned ones were also tested. Each tube was inoculated with 20,000 cells of the parasite under testing (*Entamoeba* or *Giardia*). Each compound was assayed in duplicate in each of three independent experiments. In each assay, two sets of controls were performed, one without any test compound and another with metronidazole. The caps of *Entamoeba* and *Giardia* tubes were tightly screwed and wrapped with parafilm. The tubes were incubated on a 15° horizontal slant at (36-37) °C for 72 hours.

The number of parasites in the tubes was determined as described in the literature by counting in a hemacytometer at the 10X objective. For counting of *Entamoeba* and *Giradia*, the tubes were placed on ice to permit detachment of the parasite and centrifuged at 1000 x g for 10 min. The supernatant was discarded and 1 mL fresh medium was added to each tube. Then, 25 µL of the parasite suspension in each tube was mixed with 100 µL of 0.4 % trypan blue in phosphate buffered saline (PBS) to distinguish viable from dead parasites.

### Antileishmnial activity

The antileishmanial activity of metronidazole-derivatives was tested in 96-well plates. Each of the meronidazole-derivatives and the reference drugs were dissolved in 10 µL of dimethyl sulfoxide (DMSO) and were completed with a mL growth medium. The solutions were filter sterilized using 0.22 µm syringe filters. Then, 170 µL-two fold serial dilutions of each of the compounds and metronidazole starting at a concentration of 2000 µg/mL in culture medium were prepared in flat bottomed 96-well microtiter plates of 400-µL-well-capacity. A suspension containing 10⁵ of *Leishmania* promastigotes per milliliter culture medium was prepared and 170-µL portions of the suspension were added to the appropriate wells. Plates were covered with the microtiter plate lids and incubated at 24°C. The number of parasites in each well was determined after 72 h by counting in a hemacytometer at the 10X objective lens. *Leishmania* viability was determined by observing promastigote motility. Each compound was assayed in duplicate in each of three independent experiments. In each assay two sets of controls were performed, one without any test compound and another with amphotericin B as a standard antileishmanial drug.

### MIC and IC₅₀

The minimal inhibitory concentration (MIC) defined as the minimum concentration of a compound at which no parasitic growth was observed. The 50% inhibitory concentration (IC₅₀) was the concentration of compounds which cut the number of parasites to half that in the negative control (growth medium + DMSO + parasites).

### Cytotoxicity assay

The cytotoxicity of the metronidazole-derivatives and the reference drugs was investigated on Hep-2 and Vero cells using the standard cytotoxicity assay and the trypan blue exclusion method, as described in the literature, with modifications. A cell suspension (10⁵ cells/mL RPMI medium containing 10% inactivated foetal calf serum) was prepared from confluent cultures and 100 µL portions of the suspension were added to the wells of 96-well plates. The cells were incubated for 24 h at 37 °C and 5 % CO₂ and the medium in each well was then replaced with fresh 150 µL medium. Solutions of the test compounds or reference drugs were prepared and sterilized as described above (Antiprotozolal activity). Then, 150 µL-two fold serial dilutions of each of the compounds and the reference drugs starting at a concentration of 2000 µg/mL in culture medium were prepared in the plates. After 48 hour incubation at 37 °C and 5 % CO₂, the number of cells in each well was determined as follows: The medium in each well was gently replaced with 100 µL of 0.25% (w/v) trypsin-0.53 mM EDTA solution and the plates were incubated for 5 minute at 37 °C to detach the cells followed by the addition of 200 µL of trypan blue solution were added to each well and the plates were, then, placed on ice. The cells were counted in a hemacytometer at the 10X objective. Each compound or reference drug was assayed in duplicate in each of three independent experiments. In each assay the negative controls (without any test compound or reference drug) were included in duplicates. IC₀ was the concentration of compound at which the number of cells in the well was more than or equal to that of the negative control (IC₀ = no inhibition of cell growth). The in vitro antiparasitic activity of the model compounds are given in the following Tables 1 and 2.

**Table 1. Antiamoebic and antigiardial activities of compounds IA, IIA, and IIIA**

| Compound | IC100 µg/mL | | IC₅₀ µg/mL | |
|---|---|---|---|---|
| | *Giardia intestinalis* | *Entamoeba histolytica* | *Giardia intestinalis* | *Entamoeba histolytica* |
| IA | 0.14 | 0.14 | 0.01 | 0.01 |
| IIA * | 0.3 | 0.3 | 0.04 | 0.04 |
| IIIA * | 0.8 | 0.8 | 0.04 | 0.04 |
| Metronidazole* | 3.2 | 3.2 | 0.8 | 0.8 |

| | | | | |
|---|---|---|---|---|
| *: Not of the present invention | | | | |

**Table 2. Antileishmanial activity of compounds IA, IIA, and IIIA.**

| Compound | IC100 µg/mL | | IC₅₀ µg/mL | |
|---|---|---|---|---|
| | *Leishmania donovani* | *Leishmania tropica* | *Leishmania donovani* | *Leishmania tropica* |
| IA | 2000 | 2000 | 300 | 150 |
| IIA * | 2000 | 2000 | 300 | 150 |
| IIIA * | 250 | 250 | 62.5 | 62.5 |
| Metronidazole * | ND | ND | 2000 | 2000 |
| Amphotericin B * | 2.5 | 2.5 | 0.08 | 0.08 |

| | | | | |
|---|---|---|---|---|
| ND: Not determined *: Not of the present invention | | | | |

## Claims

1. A 2-methyl-5-nitro-imidazolyl compound with the general formula I: 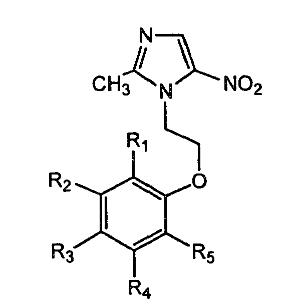 wherein:
one of the groups R₁, R₂, R₃, R₄, R₅ is an aldehyde;
and each of the other groups R₁, R₂, R₃, R₄, R₅ is independently selected from hydrogen, alkyl, alkenyl, alkoxy, amino, alkylamino, dialkylamino, aryl, aralkyl, carboxyl, carboxylic ester, cyano, amido, alkylamido and heterocyclic groups.

2. A 2-methyl-5-nitro-imidazolyl compound according to claim 1 wherein each of the other groups R₁, R₂, R₃, R₄, R₅ is independently selected from hydrogen, alkyl and alkoxy.

3. A 2-methyl-5-nitro-imidazolyl compound according to any one of the preceding claims wherein the compound has the formula IA

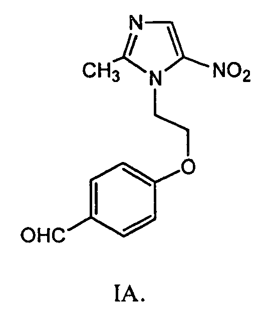

4. A 2-methyl-5-nitro-imidazolyl compound according to any one of the preceding claims for use in medicine.

5. A 2-methyl-5-nitro-imidazolyl compound according to any one of claims 1 to 3 for use in the treatment of a parasitic infection.

6. A 2-methyl-5-nitro-imidazolyl compound according to any one of claims 1 to 3 for use in the treatment of a parasitic infection caused by a parasite selected from *Entamoeba histolytica, Giardia lamblia* and pathogenic *Leishmanila* species.

7. A 2-methyl-5-nitro-imidazolyl compound according to any one of claims 1 to 3 for use in the treatment of a bacterial infection.

8. A 2-methyl-5-nitro-imidazolyl compound according to any one of claims I to 3 for use in the treatment of a bacterial infection selected from *Clostridium, Bacteroides, Helicobacter, Fusobacterium*, and *Peptostreptococcus*.

## Patentansprüche

1. 2-Methyl-5-nitroimidazolyl-Verbindung der allgemeinen Formel I: 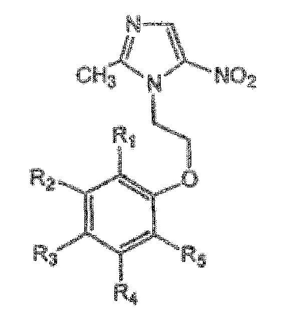 wobei:
eine der Gruppen R₁, R₂, R₃, R₄, R₅ ein Aldehyd ist
und jede der anderen Gruppen R₁, R₂, R₃, R₄, R₅ unabhängig ausgewählt ist unter Wasserstoff, Alkyl, Alkenyl, Alkoxy, Amino, Alkylamino, Dialkylanimo, Aryl, Aralkyl, Carboxyl, Carboxylester, Cyano, Amido, Alkylamido und heterocyclischen Gruppen.

2. 2-Methyl-5-nitroimidazolyl-Verbindung gemäß Anspruch 1, wobei jede der anderen Gruppen R₁, R₂, R₃, R₄, R₅ unabhängig ausgewählt ist unter Wasserstoff, Alkyl und Alkoxy.

3. 2-Methyl-5-nitroimidazolyl-Verbindung gemäß irgendeinen der vorangehenden Ansprüche, wobei die Verbindung die Formel IA hat

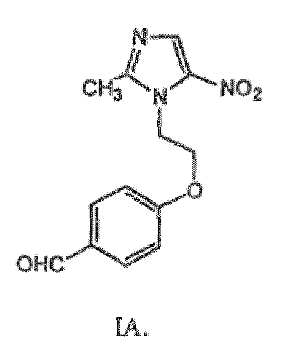

4. 2-Methyl-5-nitroimidazolyl-Verbindung gemäß irgendeinen der vorangehende Ansprüche zur Verwerdung in der Medizin.

5. 2-Methyl-5-nitroimidazolyl-Verbindung gemäß irgendeinen der Ansprüche 1 bis 3 zur Verwendung bei der Behandlung einer parasitären Infektion.

6. 2-Methyl-5-nitroimidazolyl-Verbindung gemäß irgendeinem der Ansprüche 1 bis 3 zur Verwendung bei der Behandlung einer parasitischen Infektion, verursacht durch einen Parasiten, ausgewählt unter *Entamoeba histolytica, Giardia lamblia* und pathogenen *Leishmania-Spezies.*

7. 2-Methyl-5-nitroimidazolyl-Verbindung gemäß irgendeinem der Ansprüche 1 bis 3 zur Verwerdung bei der Behandlung einer bakteriellen Infektion.

8. 2-Methyl-5-nitroimidazolyl-Verbindung gemäß irgendeinen der Ansprüche 1 bis 3 zur Verwendung bei der Behandlung einer bakteriellen Infektion, ausgewählt unter *Clostridium, Bacterioides, Helicobacter, Fusobacterium* und *Peptostreptococcus.*

## Revendications

1. Composé 2-méthyl-5-nitro-imidazolyle de formule générale I : 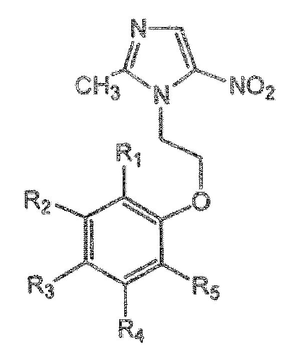 dans laquelle :
l'un des groupes R₁, R₂, R₃, R₄, R₅ est un aldéhyde ;
et chacun des autres groupes R₁, R₂, R₃, R₄, R₅ est indépendamment choisi parmi l'hydrogène et les groupes alkyle, alcényle, alcoxy, amino, alkylamino, dialkylamino, aryle, aralkyle, carboxyle, ester carboxylate, cyano, amido, alkylamido et hétérocycliques.

2. Composé 2-méthyl-5-nitro-imidazolyle selon la revendication 1, dans lequel chacun des autres groupes R₁, R₂, R₃, R₄, R₅ est indépendamment choisi parmi l'hydrogène et les groupes alkyle et alcoxy.

3. Composé 2-méthyl-5-nitro-imidazolyle selon l'une quelconque des revendications précédentes, lequel composé répond à la formule IA :

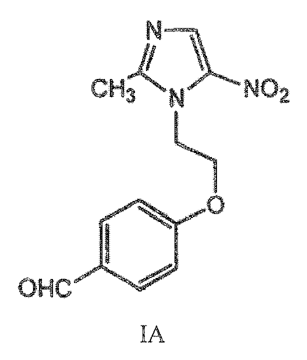

4. Composé 2-méthyl-5-nitro-imidazolyle selon l'une quelconque des revendications précédentes, pour utilisation en médecine.

5. Composé 2-méthyl-5-nitro-imidazolyle selon l'une quelconque des revendications 1 à 3, pour utilisation dans le traitement d'une infection parasitaire.

6. Composé 2-méthyl-5-nitro-imidazolyle selon l'une quelconque des revendications 1 à 3, pour utilisation dans le traitement d'une infection parasitaire causée par un parasite choisi parmi *Entamoeba histolytica, Giardia lamblia* et les espèces pathogènes de *Leishmania.*

7. Composé 2-méthyl-5-nitro-imidazolyle selon l'une quelconque des revendications 1 à 3, pour utilisation dans le traitement d'une infection bactérienne.

8. Composé 2-méthyl-5-nitro-imidazolyle selon l'une quelconque des revendications 1 à 3, pour utilisation dans le traitement d'une infection bactérienne choisie parmi *Clostridium, Bacteroides, Helicobacter, Fusobacterium,* et *Peptostreptococcus.*
